# EUROPEAN PATENT APPLICATION

(11) **EP 3 088 402 A1**
(43) Date of publication of application: **02.11.2016**
(21) Application number: 14874501.1
(22) Date of filing: 22.12.2014
(51) Int. Cl.: C07D 487/04, A61K 31/53, A61P 35/00

(54) **CRYSTALS (2) OF PYRAZINO[2,1-c][1,2,4]TRIAZINE COMPOUND**

(30) Priority: 25.12.2013 JP 2013267742
(71) Applicant: PRISM Pharma Co., Ltd., Kanagawa 226-8510 (JP)
(72) Inventor: KUSHIDA, Ikuo, Tsukuba-shi Ibaraki 300-2635 (JP); SUGAYA, Yukiko, Tsukuba-shi Ibaraki 300-2635 (JP); ODAGAMI, Takenao, Yokohama-shi Kanagawa 226-8510 (JP); KOUJI, Hiroyuki, Yokohama-shi Kanagawa 226-8510 (JP)
(74) Representative: Duckworth, Timothy John
(86) International application number: PCT/JP2014/083934
(87) International publication number: WO 2015/098855

(57) **Abstract**

Provided are a crystal of a hydrate of compound 1, showing diffraction peaks at diffraction angles (2θ±0.2°) of 10.7°, 10.9°, 14.4°, 19.1° and 22.8°, in powder X-ray diffraction, a crystal of compound 1 or hydrate thereof, showing diffraction peaks at diffraction angles (2θ±0.2°) of 7.2°, 8.9°, 13.2°, 16.4° and 22.4°, in powder X-ray diffraction, a crystal of an anhydride of compound 1, showing diffraction peaks at diffraction angles (2θ±0.2°) of 10.3°, 14.2°, 14.6°, 16.1° and 22.1°, in powder X-ray diffraction, and a crystal of compound 1 or hydrate thereof, showing diffraction peaks at diffraction angles (2θ±0.2°) of 7.3°, 10.7°, 16.3°, 19.8° and 23.8°, in powder X-ray diffraction.

## Description

### Technical Field

The present invention relates to a crystal of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide (hereinafter to be referred to as compound 1).

### Background Art

Compound 1 described in patent document 1 is a compound that inhibits the TCP4/β-Catenin transcription pathway by inhibiting CREB binding protein (CBP) in the Wnt signal transduction pathway, and is expected to treat various carcinomass (e.g., lung cancer, breast cancer, stomach cancer, pancreatic cancer, liver cancer, uterine cancer, ovarian cancer, glioma, melanoma, rectal colon cancer, lymphoma, leukemia), restenosis relating to angioplasty, angiogenesis abnormality, polycystic kidney, tuberous sclerosis, Alzheimer's disease, neurodegenerative diseases (e.g., glaucoma, macular degeneration, Parkinson's disease, Alzheimer's disease) and fibrosis (e.g., idiopathic pulmonary fibrosis).

### [Document List]

### Patent Documents

patent document 1: WO 2009/148192

### SUMMARY OF THE INVENTION

### Problems to be Solved by the Invention

In general, the property of a crystal of a compound utilizable as a pharmaceutical product exerts a marked influence on the bioavailability of a drug, purity of a drug substance, formulation of a preparation and the like.

Therefore, the problem of the present invention is to provide a crystal of compound 1 expected to be utilized as a drug substance of a pharmaceutical product.

### Means of Solving the Problems

The present inventors have conducted intensive studies in an attempt to solve the aforementioned problem and found a crystal of compound 1, and completed the present invention.

The present invention provides the following [1] - [16].
[1] A crystal of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide or hydrate thereof.
[2] A crystal of a hydrate of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide.
[3] A crystal of an anhydride of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide.
[4] The crystal of [2], showing diffraction peaks at diffraction angles (2θ±0.2°) of 10.7°, 10.9°, 14.4°, 19.1° and 22.8°, in powder X-ray diffraction.
   [4A] The crystal of [2], showing diffraction peaks at diffraction angles (2θ±0.2°) of 10.7°, 10.9°, 14.4°, 16.8°, 19.1°, 20.3°, 22.2°, 22.8°, 23.8° and 27.4°, in powder X-ray diffraction.
[5] The crystal of [1], showing diffraction peaks at diffraction angles (2θ±0.2°) of 7.2°, 8.9°, 13.2°, 16.4° and 22.4°, in powder X-ray diffraction.
   [5A] The crystal of [1], showing diffraction peaks at diffraction angles (2θ±0.2°) of 7.2°, 8.9°, 10.9°, 12.9°, 13.2°, 16.4°, 17.1°, 18.4°, 22.4° and 24.8°, in powder X-ray diffraction.
[6] The crystal of [3], showing diffraction peaks at diffraction angles (2θ±0.2°) of 10.3°, 14.2°, 14.6°, 16.1° and 22.1°, in powder X-ray diffraction.
   [6A] The crystal of [3], showing diffraction peaks at diffraction angles (2θ±0.2°) of 10.3°, 12.8°, 14.2°, 14.6°, 16.1°, 18.9°, 20.4°, 21.6°, 22.1° and 24.1°, in powder X-ray diffraction.
[7] The crystal of [1], showing diffraction peaks at diffraction angles (2θ±0.2°) of 7.3°, 10.7°, 16.3°, 19.8° and 23.8°, in powder X-ray diffraction.
   [7A] The crystal of [1], showing diffraction peaks at diffraction angles (2θ±0.2°) of 7.3°, 9.9°, 10.7°, 12.2°, 15.4°, 16.3°, 17.7°, 19.8°, 23.1°, 23.8° and 25.0°, in powder X-ray diffraction.
[8] A pharmaceutical composition comprising the crystal of any of [1] - [7A] as an active ingredient.
[9] An antitumor agent comprising the crystal of any of [1] - [7A] as an active ingredient.
[10] A method of preventing or treating tumor, comprising administering a pharmacologically effective amount of any of the crystals of [1] - [7A] to a patient.
[11] The crystal of any of [1] - [7A], which is used for the prophylaxis or treatment of tumor.
[12] Use of the crystal of any of [1] - [7A] in the production of an antitumor agent.

### Effect of the Invention

The crystal of compound 1 provided by the present invention has good property as a drug substance of a pharmaceutical product.

### Brief Description of the Drawings

Fig. 1 shows a powder X-ray diffraction pattern of crystal A of compound 1 obtained in Example 1, wherein the horizontal axis shows diffraction angle (2θ), and the vertical axis shows peak intensity.
Fig. 2 shows a powder X-ray diffraction pattern of crystal B of compound 1 obtained in Example 2, wherein the horizontal axis shows diffraction angle (2θ), and the vertical axis shows peak intensity.
Fig. 3 shows a powder X-ray diffraction pattern of crystal C of compound 1 obtained in Example 3, wherein the horizontal axis shows diffraction angle (2θ), and the vertical axis shows peak intensity.
Fig. 4 shows a powder X-ray diffraction pattern of crystal D of compound 1 obtained in Example 4, wherein the horizontal axis shows diffraction angle (2θ), and the vertical axis shows peak intensity.
Fig. 5 shows a powder X-ray diffraction pattern of an amorphous form of compound 1 obtained in Comparative Example 1, wherein the horizontal axis shows diffraction angle (2θ), and the vertical axis shows peak intensity.
Fig. 6 is a graph showing the hygroscopicity of crystal A of compound 1 obtained in Example 1, wherein the horizontal axis shows relative humidity (RH), and the vertical axis shows variation (%) in the mass.
Fig. 7 is a graph showing the hygroscopicity of crystal C of compound 1 obtained in Example 3, wherein the horizontal axis shows relative humidity (RH), and the vertical axis shows variation (%) in the mass.
Fig. 8 is a graph showing the hygroscopicity of compound 1 obtained in Comparative Example 1, wherein the horizontal axis shows relative humidity (RH), and the vertical axis shows variation (%) in the mass.

### Description of Embodiments

Preferable crystals in the present specification include
a crystal of a hydrate of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 10.7°, 10.9°, 14.4°, 19.1° and 22.8°, in powder X-ray diffraction;
a crystal of a hydrate of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 10.7°, 10.9°, 14.4°, 16.8°, 19.1°, 20.3°, 22.2°, 22.8°, 23.8° and 27.4°, in powder X-ray diffraction;
a crystal of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 7.2°, 8.9°, 13.2°, 16.4° and 22.4°, in powder X-ray diffraction;
a crystal of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 7.2°, 8.9°, 10.9°, 12.9°, 13.2°, 16.4°, 17.1°, 18.4°, 22.4° and 24.8°, in powder X-ray diffraction;
a crystal of an anhydride of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 10.3°, 14.2°, 14.6°, 16.1° and 22.1°, in powder X-ray diffraction;
a crystal of an anhydride of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 10.3°, 12.8°, 14.2°, 14.6°, 16.1°, 18.9°, 20.4°, 21.6°, 22.1° and 24.1°, in powder X-ray diffraction;
a crystal of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 7.3°, 10.7°, 16.3°, 19.8° and 23.8°, in powder X-ray diffraction;
a crystal of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide, showing diffraction peaks at diffraction angles (2θ±0.2°) of 7.3°, 9.9°, 10.7°, 12.2°, 15.4°, 16.3°, 17.7°, 19.8°, 23.1°, 23.8° and 25.0°, in powder X-ray diffraction, and the like.

In general, the diffraction angle (2θ) in powder X-ray diffraction produces an error within the range of +0.2°. Therefore, the value of the above-mentioned diffraction angle needs to be understood to include numerical values within the range of about +0.2°. Accordingly, not only the crystals showing peaks at completely identical diffraction angles in powder X-ray diffraction, but also crystals showing peaks at diffraction angles different only by about +0.2° are also encompassed in the present invention.

Therefore, in the present specification, for example, "showing a diffraction peak at a diffraction angle (2θ±0.2°) of 10.5°" means that it "shows a diffraction peak at a diffraction angle (2θ) of 10.3° - 10.7°", and the same applies to other diffraction angles.

The production method of the crystal of compound 1 is explained below.

Unless otherwise specified, the water content in the present specification is a numerical value measured based on the Karl Fischer method.

### production method of a crystal of compound 1 or hydrate thereof

A crystal of compound 1 can be obtained by a general crystallization method and using compound 1 or a salt thereof. Examples of the general crystallization method include a method of crystallizing a slurry of compound 1 added with a solvent, a method including dissolving compound 1 in a solvent and adding a poor solvent (solvent showing low solubility of compound 1) to performed crystallization, a method including dissolving compound 1 in a solvent with heating, and gradually cooling the mixture with stirring to perform crystallization, and a method including dissolving compound 1 in a solvent, concentrating and standing the mixture, or gradually concentrating with stirring to perform crystallization.

Compound 1 or a salt thereof to be used for crystallization may have any form, may be a solvate or an anhydride, may be amorphous or crystalline (including one composed of plural polycrystallines), and may be a mixture of these. The solvate includes hydrate, methanolate and the like.

The solvent used for crystallization includes, for example, alcohol solvents such as methanol, ethanol, isopropanol, 1-propanol and the like; acetonitrile; dimethyl sulfoxide (DMSO); amide solvents such as N,N-dimethylformamide (DMF) and the like; ester solvents such as methyl acetate, ethyl acetate, isopropyl acetate and the like; saturated hydrocarbon solvents such as hexane, heptane and the like; ketone solvents such as acetone, methylethyl ketone and the like; ether solvents such as t-butyl methyl ether (hereinafter to be referred to as MTBE), tetrahydrofuran (THF) and the like; dichloromethane and water. These solvents may be used alone or a mixture of two or more kinds thereof may be used. Preferred is a mixed solvent of alcohol solvent and water.

The amount of the solvent to be used can be appropriately selected wherein an amount capable of dissolving compound 1 or a salt thereof by heating or an amount that enables stirring of a suspension obtained by adding a solvent to compound 1 or a salt thereof is the lower limit, and an amount free of a marked decrease in the yield of crystal is the upper limit.

In crystallization, a seed crystal (crystal of desired compound 1 and the like) may or may not be added. While the temperature at which a seed crystal is added is not particularly limited, it is preferably 0°C - 60°C.

As the temperature at which compound 1 or a salt thereof is dissolved by heating, a temperature at which compound 1 is dissolved can be appropriately selected according to the solvent. Preferred is a temperature range from room temperature to a temperature at which a solvent used for crystallization starts refluxing, and more preferred is room temperature - 100°C.

Since cooling during crystallization may afford a crystal with different form (polymorphism) when the cooling is rapid. Therefore, cooling is desirably performed after appropriately adjusting the cooling rate in consideration of the quality of crystal and influence on the particle size and the like. Cooling at 5°C - 40°C/h is preferable, and cooling at a rate of 5°C - 25°C/h is more preferable.

While the final crystallization temperature can be appropriately selected according to the desired object such as yield, quality and the like of the crystal, it is preferably-25°C - 30°C.

The object crystal can be obtained by separating the crystal obtained by crystallization by a general filtration operation, washing the filtered crystal with a solvent as necessary, and drying same. As the solvent to be used for washing the crystal, those similar to the solvents used for crystallization can be used. Preferred are, for example, ethanol, acetone, ethyl acetate, isopropyl acetate, and MTBE.

The crystal separated by the filtration operation can be dried by leaving in the air or a nitrogen stream as appropriate, or by heating.

The drying time can be selected as appropriate as the time necessary for the residual solvent to become less than a given amount, and according to the production amount, dryer, drying temperature and the like. Drying can be performed under ventilation or under reduced pressure. The depressurization. level can be appropriately selected according to the production amount, dryer, drying temperature and the like. The obtained crystal can also be left in the air as necessary after drying.

The above-mentioned crystal can also be produced by, in the above-mentioned production method of compound 1, performing the production method of the crystal of compound 1 sequentially after the synthesis of compound 1.

The crystal of compound 1 can be formulated according to a conventional method. Examples of the dosage form of formulation include oral preparation (tablet, granule, powder, capsule, syrup etc.), injection (for intravenous administration, intramuscular administration, subcutaneous administration, intraperitoneal administration etc.), and external preparation (transdermal absorption preparation (ointment, adhesive preparation etc.), eye drop, nasal drop, suppository etc.).

When an oral solid preparation (tablet, granule, powder, capsule etc.) is produced, additives such as excipient, binder, disintegrant, lubricant, colorant and the like are added as necessary to the crystal of compound 1, and an oral solid preparation is produced by a conventional method. When an oral solid preparation is produced, coating may be applied as necessary.

Examples of the excipient include lactose, cornstarch, crystalline cellulose and the like, and examples of the binder include hydroxypropylcellulose, hydroxypropylmethylcellulose and the like. Examples of the disintegrant include calcium carboxymethylcellulose, croscarmellose sodium and the like, and examples of the lubricant include magnesium stearate, calcium stearate and the like. Examples of the colorant include titanium oxide and the like, and examples of the coating agent include hydroxypropylcellulose, hydroxypropylmethylcellulose, methylcellulose and the like. Usable additives are not limited to these.

These oral solid preparations can generally contain 0.001 - 99.5 wt%, preferably 0.01 - 90 wt% and the like, of the crystal of compound 1.

When an injection (for intravenous administration, intramuscular administration, subcutaneous administration, intraperitoneal administration etc.) is produced, pH adjuster, buffering agent, suspending agent, solubilizing agent, antioxidant, preservative (antiseptic), isotonicity agent and the like are added as necessary to the crystal of compound 1, and injection can be produced by a conventional method. The injection may be formed as a use-time dissolution type freeze-dry preparation by freeze-drying.

As the pH adjuster and buffering agent, organic acid or inorganic acid and/or a salt thereof and the like can be used. As the suspending agent, methylcellulose, polysorbate 80, sodium carboxymethylcellulose and the like can be used; as the solubilizing agent, polysorbate 80, polyoxyethylene sorbitan monolaurate and the like can be used. As the antioxidant, α-tocopherol and the like can be used; as the preservative, methyl p-hydroxybenzoate, ethyl parahydroxybenzoate and the like can be used; as the isotonicity agent, glucose, sodium chloride, mannitol and the like can be used. The pH adjuster, buffering agent, suspending agent, solubilizing agent, antioxidant, preservative (antiseptic), and isotonicity agent are not limited to these.

These injections can generally contain 0.000001 - 99.5 mass %, preferably 0.00001 - 90 mass % and the like of the crystal of compound 1 relative to the total mass of the injection.

When an external preparation is produced, a base starting material is added to the crystal of compound 1 and, for example, preservative, stabilizer, pH adjuster, antioxidant, colorant and the like are added as necessary, and an external preparation can be produced by a conventional method.

As the base starting material to be used, various starting materials generally used for pharmaceutical products, quasi-drugs, cosmetics and the like can be used. Specifically, for example, starting materials such as animal and vegetable oils, mineral oil, ester oil, waxes, emulsifier, higher alcohols, fatty acids, silicon oil, surfactant, phospholipids, alcohols, polyvalent alcohols, water-soluble polymers, clay minerals, purified water and the like can be mentioned.

These external preparations can generally contain 0.000001 - 99.5 wt%, preferably 0.00001 - 90 wt% and the like of the crystal of compound 1.

While the dose of the crystal of compound 1 varies depending on the level of symptom, age, sex, body weight, administration form, kind of salt, specific kind of the disease and the like, in the case of an adult, generally about 30 µg - 10 g, preferably 100 µg - 5 g, further preferably 100 µg - 1 g, is administered per day by oral administration, and about 30 µg - 1 g, preferably 100 µg - 500 mg, further preferably 100 µg - 300 mg, is administered per day by injection administration, each in one to several portions.

### Examples

The present invention is explained in detail in the following by referring to Reference Examples and Examples, which are not to be construed as limitative. The "room temperature" is a temperature within the range of 1 - 30°C.

In the following, 4 kinds of crystals are explained as crystals A, B, C and D. However, the crystal of compound 1 of the present invention is not limited to them.

Crystal A is a crystal of a hydrate of compound 1 and has a melting point of 112°C. Crystal A can be obtained, for example, by crystallizing compound 1 from a mixed solvent of an alcohol solvent such as methanol, ethanol, isopropanol and the like and water.

While crystal A is not transited by heating at 50°C overnight, it transits to an amorphous form by heating at 70°C overnight.

Crystal B is a crystal having a melting point of 140°C. Crystal B can be obtained, for example, by crystallizing compound 1 from methanol. It can also be obtained by adding methanol to crystal A and leaving the mixture at room temperature for 2 days.

Crystal C is a crystal of an anhydride of compound 1 and has a melting point of 187°C. Crystal C can be obtained, for example, by crystallizing compound 1 from a mixed solvent of ethanol, isopropanol or ethyl acetate, and heptane. It can also be obtained by dissolving compound 1 in isopropyl acetate, and crystallizing same by heating under reduced pressure at 40°C. While crystal C is transited to crystal D by adding a mixed solvent of heptane and ethanol (volume ratio 16:3) which has a water content of not less than 0.5% and leaving the mixture overnight, it is not transited by adding a mixed solvent having a water content of less than 0.5% and leaving the mixture overnight.

Crystal D is a crystal having a melting point of 105°C. It can be obtained, for example, by adding a mixed solvent of heptane and ethanol (volume ratio 16:3) which has a water content of not less than 0.5% to crystal C and leaving the mixture overnight.

A part of crystal D transits to an amorphous form by heating at 50°C overnight, and crystal D transits to an amorphous form by heating at 70°C overnight. In addition, crystal D transits to crystal C by adding a mixed solvent of heptane and ethanol (volume ratio 16:3) which has a water content of less than 0.5% and leaving the mixture overnight.

The chemical shift of ¹H-NMR (proton nuclear magnetic resonance) spectrum was recorded in δ unit (ppm) to tetramethylsilane, and the coupling constant was recorded in hertz (Hz). The abbreviations of splitting patterns are as follows. s: singlet, d: doublet, t: triplet, q: quartette, m: multiplet, brs: broad singlet, brd: broad doublet.

### Comparative Example 1

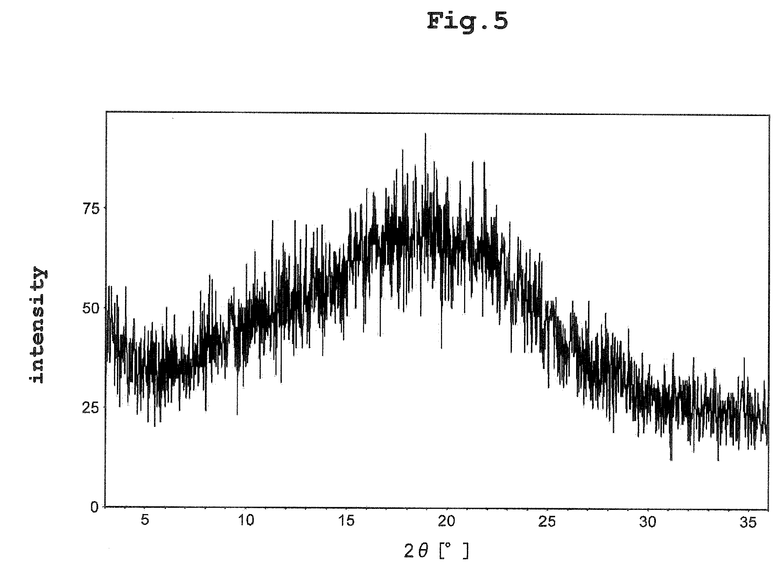

According to the production method described in patent document 1, compound 1 was obtained as a solid. The powder X-ray diffraction of the obtained solid was measured, and the results thereof are shown in Fig. 5. From Fig. 5, it was confirm that compound 1 obtained in Comparative Example 1 was amorphous.
¹H-NMR (600 MHz, CHLOROFORM-d) δ (ppm): 1.27 (d, J=6 Hz, 3H), 2.59 (s, 3H), 3.01 (d, J=17 Hz, 1H), 3.24 (dd, J=14, 9 Hz, 1H), 3.35 (d, J=17 Hz, 1H), 3.43 (dd, J=14, 5 Hz, 1H), 4.03 (m, 1H), 4.32 (dd, J=15, 6 Hz, 1H), 4.51 (dd, J=15, 7 Hz, 1H), 5.35 (d, J=16 Hz, 1H), 5.50 (d, J=16 Hz, 1H), 5.56 (dd, J=9, 5 Hz, 1H), 5.83 (d, J=10 Hz, 1H), 6.16 (br. s, 1H), 6.63 (d, J=8 Hz, 2H), 6.90 (t, J=6 Hz, 1H), 7.07 (d, J=8 Hz, 2H), 7.26 (d, J=8 Hz, 2H), 7.30 (t, J=7 Hz, 1H), 7.36 (dd, J=8, 7 Hz, 2H), 7.41 (dd, J=8, 4 Hz, 1H), 7.52 (t, J=8 Hz, 1H), 7.61 (d, J=7 Hz, 1H), 7.75 (d, J=8 Hz, 1H), 8.16 (dd, J=8, 2 Hz, 1H), 8.88 (dd, J=4, 2 Hz, 1H).

### Example 1

### Preparation of crystal of compound 1 (crystal A)

Water (2 mL) was added to compound 1 (303 mg) obtained in Comparative Example 1, and isopropanol (0.52 mL) and ethanol (0.5 mL) were added with heat-stirring. As a result, it was completely dissolved at about 93°C. The mixture was allowed to slowly cool to room temperature, and precipitation of a solid was confirmed. The precipitate was collected by filtration through a glass filter, washed with heptane, and air dried at 60°C to give the title crystal (221 mg) as a white solid.

### Example 2

### Preparation of crystal of compound 1 (crystal B)

Compound 1 (100 mg) obtained in Example 1 was dissolved in methanol (400 mg), and the mixture was stirred at room temperature for 5 - 10 hr. The precipitated solid was collected by filtration and dried to give the title crystal.

### Example 3

### Preparation of crystal of compound 1 (crystal C)

Ethyl acetate (1 mL) was added to compound 1 (300 mg) obtained in Comparative Example 1 and the mixture was heat-stirred. As a result, it was completely dissolved at about 68°C. When heating was stopped, a solid was precipitated at about 66°C, and the mixture was allowed to slowly cool to room temperature. The precipitate was collected by filtration through a glass filter, washed with heptane, and air dried at 60°C to give the title crystal (228 mg) as a white solid.

### Example 4

### Preparation of crystal of compound 1 (crystal D)

Water (14 mg) was added to ethanol (240 mg) and heptane (1100 mg), and the mixture was stirred. Compound 1 (100 mg) obtained in Example 3 was added to the obtained mixed solvent, and the mixture was stirred at 5 - 25°C for 15 hr. The precipitated solid was collected by filtration and dried to give the title crystal.

### Experimental Example 1: powder X-ray diffraction measurement

For powder X-ray diffraction measurement, crystals A - D were placed on a sample table of a powder X-ray diffraction apparatus, and measurement was performed under the following conditions.

### (measurement conditions)

apparatus: Rigaku MiniFlex II Desktop X-ray Diffractometer
X-ray used: Cu Kα ray
detector: scintillation counter
tube voltage: 30 kV
tube electric current: 15 mA
Kβ filter: nickel filter
divergence slit: 1.25°
scattering slit: 1.25°
receiving slit: 0.3 mm
soller slit: 5° (divergence angle)
scan rate: 5°/min
sampling interval: 0.02°
scan range: 3° - 36°
sample holder: aluminum holder

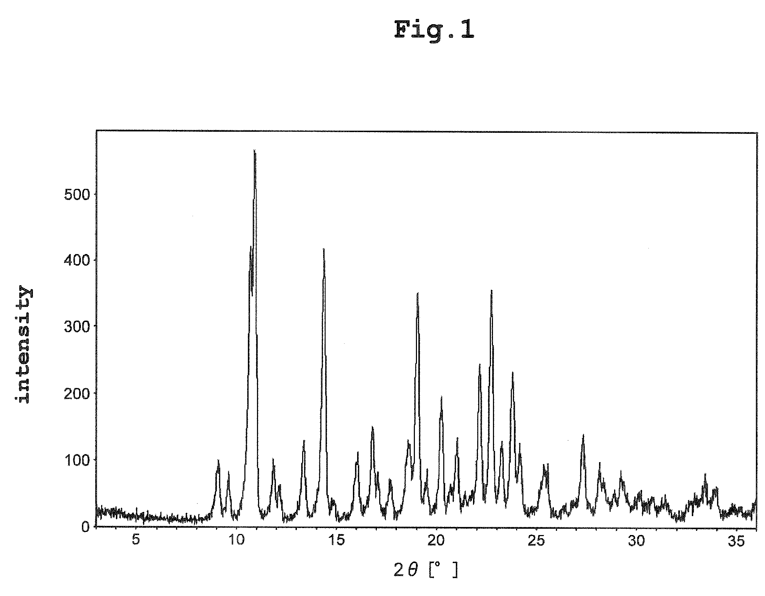

As a result of the powder X-ray diffraction measurement of crystals A, B, C and D, diffraction patterns shown in Figs. 1 - 4 were obtained.

The powder X-ray diffraction of crystal A obtained in Example 1 was measured, and the diffraction pattern shown in Fig. 1 and Table 1 was obtained.

**Table 1**

| 2θ [°] | peak intensity [counts] |
|---|---|
| 9.1 | 87 |
| 9.6 | 67 |
| 10.7 | 404 |
| 10.9 | 550 |
| 11.9 | 87 |
| 12.2 | 48 |
| 13.4 | 113 |
| 14.4 | 401 |
| 16.1 | 91 |
| 16.8 | 127 |
| 17.1 | 62 |
| 17.7 | 49 |
| 18.6 | 112 |
| 19.1 | 330 |
| 19.6 | 53 |
| 20.3 | 167 |
| 20.7 | 33 |
| 21.1 | 102 |
| 22.2 | 205 |
| 22.8 | 313 |
| 23.3 | 88 |
| 23.8 | 199 |
| 24.2 | 97 |
| 25.4 | 66 |
| 27.4 | 118 |
| 28.2 | 72 |
| 28.9 | 29 |
| 29.3 | 42 |
| 30.2 | 27 |
| 33.5 | 59 |
| 34.0 | 32 |

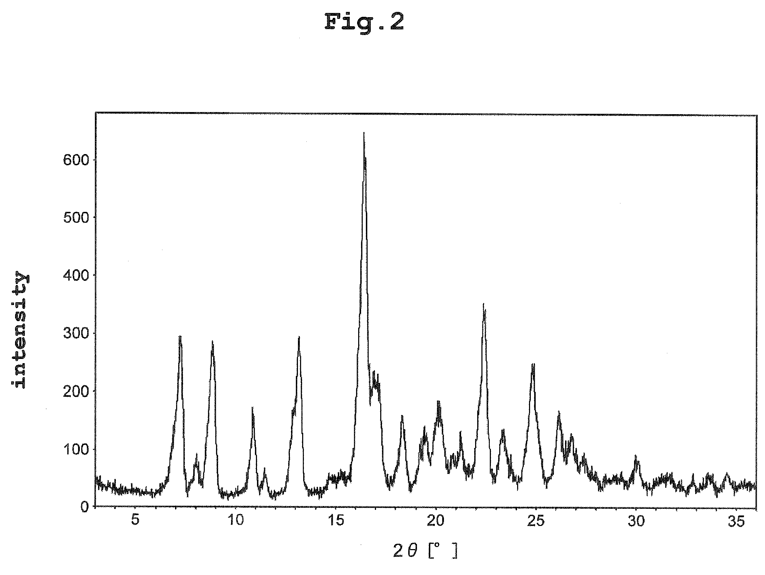

The powder X-ray diffraction of crystal B obtained in Example 2 was measured, and the diffraction pattern shown in Fig. 2 and Table 2 was obtained.

**Table 2**

| 2θ [°] | peak intensity [counts) |
|---|---|
| 7.2 | 266 |
| 8.1 | 54 |
| 8.9 | 268 |
| 10.9 | 139 |
| 11.5 | 27 |
| 12.9 | 149 |
| 13.2 | 271 |
| 16.4 | 592 |
| 17.1 | 189 |
| 18.4 | 121 |
| 19.6 | 95 |
| 20.2 | 109 |
| 21.3 | 59 |
| 22.4 | 297 |
| 23.4 | 87 |
| 24.8 | 203 |
| 26.2 | 113 |
| 26.8 | 74 |
| 27.4 | 32 |
| 30.1 | 44 |
| 32.9 | 29 |
| 34.6 | 28 |

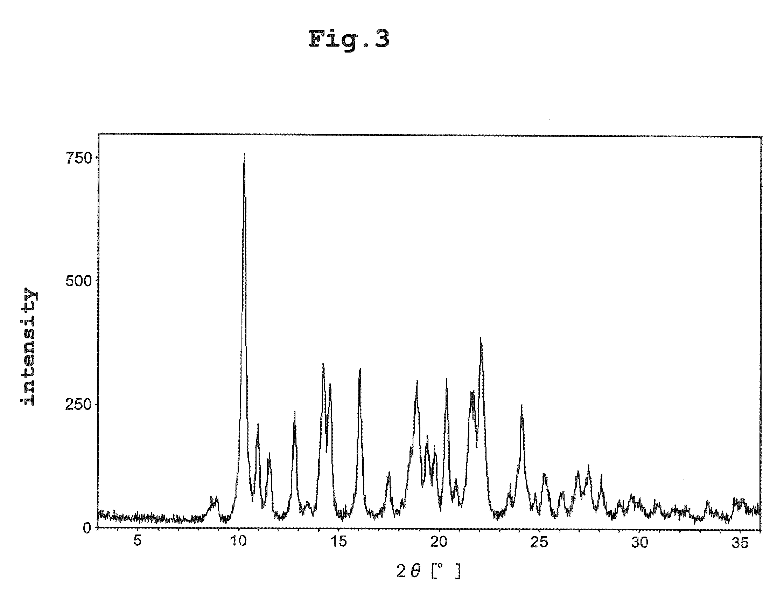

The powder X-ray diffraction of crystal C obtained in Example 3 was measured, and the diffraction pattern shown in Fig. 3 and Table 3 was obtained.

**Table 3**

| 2θ [°] | peak intensity [counts) |
|---|---|
| 9.0 | 41 |
| 10.3 | 745 |
| 11.0 | 186 |
| 11.6 | 130 |
| 12.8 | 211 |
| 14.2 | 305 |
| 14.6 | 265 |
| 16.1 | 300 |
| 17.5 | 85 |
| 18.6 | 135 |
| 18.9 | 263 |
| 19.4 | 120 |
| 19.8 | 96 |
| 20.4 | 248 |
| 20.9 | 48 |
| 21.6 | 228 |
| 22.1 | 348 |
| 23.6 | 55 |
| 24.1 | 218 |
| 25.2 | 78 |
| 26.2 | 41 |
| 27.0 | 81 |
| 27.5 | 95 |
| 28.1 | 76 |
| 29.0 | 26 |
| 29.6 | 43 |
| 31.0 | 24 |
| 33.4 | 36 |
| 34.8 | 32 |
| 35.2 | 30 |

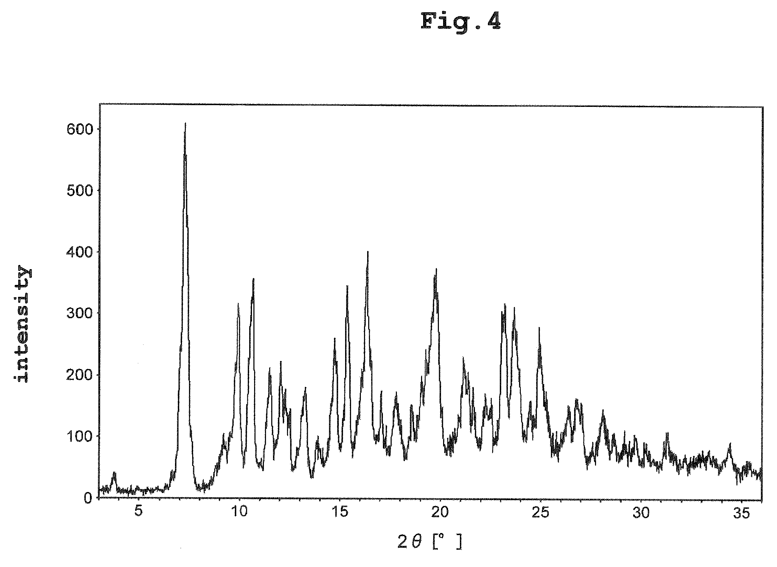

The powder X-ray diffraction of crystal D obtained in Example 4 was measured, and the diffraction pattern shown in Fig. 4 and Table 4 was obtained.

**Table 4**

| 2θ [°] | peak intensity [counts) |
|---|---|
| 3.8 | 29 |
| 7.3 | 396 |
| 9.2 | 96 |
| 9.9 | 112 |
| 10.7 | 267 |
| 11.5 | 139 |
| 12.2 | 174 |
| 13.2 | 147 |
| 14.7 | 99 |
| 15.4 | 160 |
| 16.3 | 249 |
| 17.7 | 164 |
| 19.0 | 80 |
| 19.8 | 284 |
| 21.2 | 151 |
| 21.7 | 113 |
| 22.3 | 135 |
| 23.1 | 212 |
| 23.8 | 283 |
| 24.6 | 68 |
| 25.0 | 213 |
| 26.4 | 95 |
| 26.9 | 58 |
| 28.1 | 97 |
| 29.8 | 51 |
| 33.1 | 74 |
| 34.4 | 35 |

### Experimental Example 2: hygroscopicity evaluation

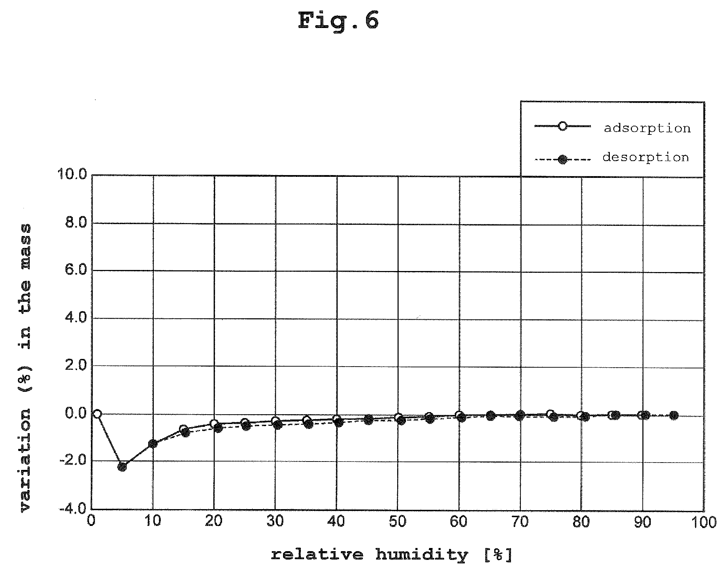

The hygroscopicity of Examples 1, 3 and Comparative Example 1 was evaluated by a dynamic water adsorption measuring apparatus. A sample mounting part of the apparatus was maintained at 25°C, and the relative humidity (RH) was set stepwisely within the range of 5 - 95%RH. The humidity was controlled by changing the relative flow of 0%RH dry nitrogen and 100%RH humidified nitrogen. The sample weight was confirmed at 2 min intervals by a micro balance, and the humidity was sequentially changed at the time point when the weight shift range for 5 min was below 0.01%. The results thereof are shown in Figs. 6 - 8.

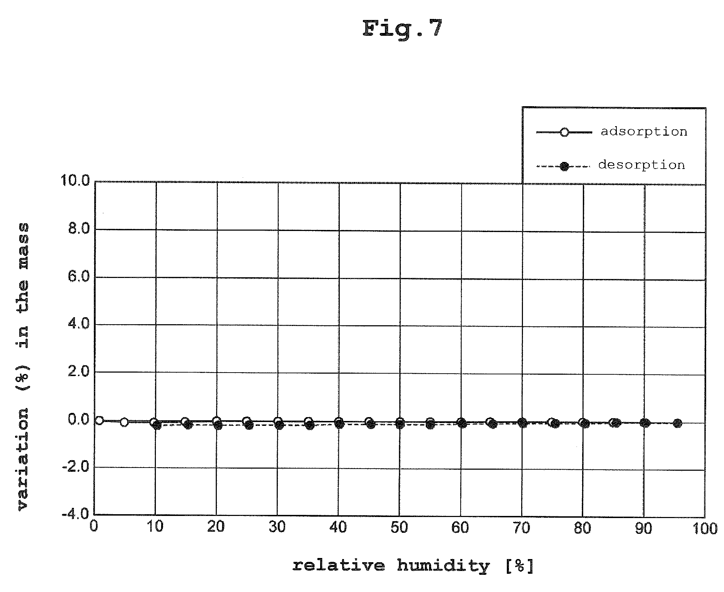

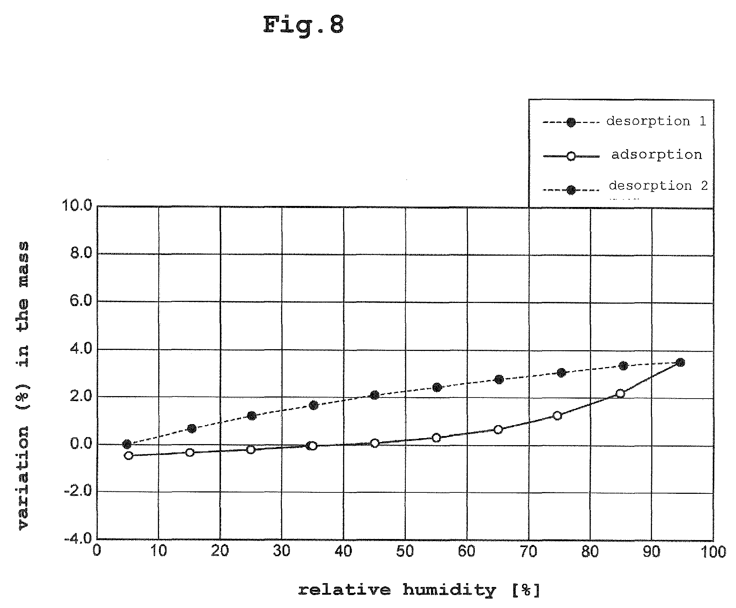

In Comparative Example 1, the weight increased along with an increase in the humidity, as shown in Fig. 8. In contrast, the weight did not increase in Examples 1 and 3 even when the humidity increased, as shown in Fig. 6 and Fig. 7, respectively. Therefore, it was clarified that the hygroscopicity of crystals A and C is low.

### Experimental Example 3: stability evaluation of crystal A

Crystal A was added in excess to the solvents described in Table 1 to give a slurry, which was stood at room temperature for 2 days, and the crystal form thereof was measured using powder X-ray diffraction and differential scanning calorimeter (DSC). The results thereof are shown in Table 5.

**Table 5**

| | solvent | crystal form |
|---|---|---|
| 1 | ethanol | crystal C |
| 2 | ethyl acetate | mixture of crystal A and crystal C |
| 3 | isopropanol | crystal C |
| 4 | isopropyl acetate | crystal A |
| 5 | MTBE | crystal A |
| 6 | methanol | crystal B |
| 7 | water | crystal A |
| 8 | tetrahydrofuran | amorphous |
| 9 | acetonitrile | crystal A |
| 10 | methylethyl ketone | crystal A |

When isopropyl acetate, MTBE, acetonitrile, methylethyl ketone or water was added to crystal A and the mixture was stood at room temperature for 2 days, transition did not occur. When methanol was added to crystal A and the mixture was stood at room temperature for 2 days, transition to crystal B occurred. When ethanol, isopropanol or ethyl acetate was added and the mixture was stood at room temperature for 2 days, transition to crystal C occurred gradually. When tetrahydrofuran was added to crystal A and the mixture was stood at room temperature for 2 days, transition to an amorphous form occurred.

### Experimental Example 4: relationship between water content of solvent and crystal form

To a saturated solution of crystal C in a mixed solvent of heptane and ethanol (volume ratio16:3) were added crystal C (100 mg) and crystal D (100 mg) at a temperature shown in Table 2, water in the amount shown in Table 6 was further added, and the mixture was stood overnight. The water content of the solution after the standing was measured by the Karl Fischer method, and the crystal form of compound 1 was measured by powder X-ray diffraction. The results thereof are shown in Table 6. The amount of the water added is shown in mass % relative to the mass of the whole solvent.

**Table 6**

| | temperature (°C) | amount of water added (mass%) | water content (%) | crystal form |
|---|---|---|---|---|
| 1 | 5 | 0 | 0.32 | crystal C |
| 2 | room temperature | 0 | 0.33 | crystal C |
| 3 | 40 | 0 | 0.33 | crystal C |
| 4 | 5 | 0.5 | 0.93 | crystal D |
| 5 | room temperature | 0.5 | 0.93 | crystal D |
| 6 | 40 | 0.5 | 0.94 | crystal D |
| 7 | 5 | 1.0 | 1.56 | crystal D |
| 8 | room temperature | 1.0 | 1.57 | crystal D |
| 9 | 40 | 1.0 | 1.55 | crystal D |

When the water content was less than 0.5%, crystal C was obtained, whereas when the water content was not less than 0.5%, crystal D was obtained. When the amount of water to be further added exceeded 2 mass %, crystal A was obtained. When crystal D was added to a mixed solvent of heptane and ethanol (volume ratio 16:3) which has a water content of less than 0.5% and the mixture was stood, transition to crystal C occurred. When crystal C was added to a mixed solvent of heptane and ethanol (volume ratio 16:3) which has a water content of less than 0.5% and the mixture was stood, transition of crystal form did not occur.

### Experimental Example 5: thermal stability of crystals A and D

Crystals A and D were heated in an oven at 50°C or 70°C overnight, and transition of each crystal was confirmed by powder X-ray diffraction.

**[Table 7]**

| | temperature | crystals used | |
|---|---|---|---|
| | | crystal A | crystal D |
| 1 | 50°C | crystal A | mixture of crystal D and amorphous form |
| 2 | 70°C | amorphous | amorphous |

As shown in Table 7, crystal A did not show transition under heating conditions at 50°C, whereas a part of crystal D transited to an amorphous form. Therefore, crystal A was more stable to heat than crystal D.

### Experimental Example 6: stability evaluation of crystals A, B and C

To a supersaturated solution of compound 1 using the solvents shown in Table 8 was added a mixture of equal amounts of crystals A, B and C at room temperature or 40°C, and the form of the crystal obtained one day later was observed using powder X-ray diffraction. The results thereof are shown in Table 8.

**[Table 8]**

| | solvent | temperature | |
|---|---|---|---|
| | | room temperature | 40°C |
| 1 | isopropanol | crystal C | crystal C |
| 2 | isopropyl acetate | crystal C | crystal C |
| 3 | MTBE | crystal C | crystal C |
| 4 | ethyl acetate | crystal C | crystal C |
| 5 | water | crystal A | crystal A |

As shown in Table 8, when water was used as the solvent, crystal A was obtained; when isopropanol, isopropyl acetate, MTBE or ethyl acetate was used as the solvent, crystal C was obtained. Therefore, of crystals A, B and C, crystal A was most stable in water, and crystal C was most stable in other nonaqueous solvents.

## Claims

1. A crystal of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide or hydrate thereof.

2. A crystal of a hydrate of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide.

3. A crystal of an anhydride of (6S,9S)-N-benzyl-6-(4-hydroxybenzyl)-2,9-dimethyl-4,7-dioxo-8-(quinolin-8-ylmethyl)octahydro-1H-pyrazino[2,1-c][1,2,4]triazine-1-carboxamide.

4. The crystal according to claim 2, showing diffraction peaks at diffraction angles (2θ±0.2°) of 10.7°, 10.9°, 14.4°, 19.1° and 22.8°, in powder X-ray diffraction.

5. The crystal according to claim 1, showing diffraction peaks at diffraction angles (2θ±0.2°) of 7.2°, 8.9°, 13.2°, 16.4° and 22.4°, in powder X-ray diffraction.

6. The crystal according to claim 3, showing diffraction peaks at diffraction angles (2θ±0.2°) of 10.3°, 14.2°, 14.6°, 16.1° and 22.1°, in powder X-ray diffraction.

7. The crystal according to claim 1, showing diffraction peaks at diffraction angles (2θ±0.2°) of 7.3°, 10.7°, 16.3°, 19.8° and 23.8°, in powder X-ray diffraction.

8. A pharmaceutical composition comprising the crystal according to any one of claims 1 to 7 as an active ingredient.

9. An antitumor agent comprising the crystal according to any one of claims 1 to 7 as an active ingredient.
